# EUROPEAN PATENT APPLICATION

(11) **EP 1 604 657 A1**
(43) Date of publication of application: **14.12.2005**
(21) Application number: 04425739.2
(22) Date of filing: 04.10.2004
(51) Int. Cl.: A61K 31/167, A61K 47/12, A61K 47/10, A61K 9/12

(54) **Pharmaceutical formulation of a stable Formoterol-containing solution for use in inhalation therapy and method of preparing it**

(30) Priority: 31.05.2004 IT RM20040268
(71) Applicant: Italchimici SpA, 00040 Pomezia (IT)
(72) Inventor: Sonaglia, Achille, 00040 Pomezia (IT); Annaloro, Raffaele, 00040 Pomezia (IT)
(74) Representative: Conforti Cioncoloni, Marisa

(57) **Abstract**

A pharmaceutical formulation of a Formoterol-containing water-propylene glycol solution is composed of Formoterol, as an active substance, in a concentration variable between 0.001 and 0.06 per cent, a stabilizer of citric acid and sodium citrate, in an amount variable between 0.005 and 0.5 per cent, and a solvent of propylene glycol and water, in the required amount to achieve 100 per cent of a finished solution. A method of preparing such a solution is also described.

## Description

The present invention relates to a pharmaceutical formulation of a stable Formoterol-containing solution, without adding any preservative, for use in inhalation therapy. Further it concerns a method of preparing such a solution in the form of monodose glass phials, so as to allow a simple and ready administration by electrical nebulizers usually available on the market.

Formoterol is a selective antagonist of pulmonary beta2-adrenergic receptors. It is provided with a quickly starting, strong bronchodilation activity, whose effect lasts also for 12 hours from the administration thereof. The inhalation administration is favourite, as it permits the active substance to reach directly the field of action, permits the doctor to use lower daily dosages, and causes less systemic collateral effects in a patient, with respect to the general administration.

Nowdays Formoterol is used in the therapy of the bronchial asthma as powder for inhalation or pre-doser inhalator, and needs to be cold preserved due its high instability.

In the prior art there are patents to Boehringer Ingelheim Pharma (e.g., US-6,150,418 granted on November 21, 2000 and EA3960 published on October 30, 2003), that disclose Formoterol stored in solutions and suspensions. However, as for example in US-6,150,418, in these solutions and suspensions Formoterol as an active substance is concentrated (between about 75 mg/ml and about 500 mg/ml).

These patents do not teach how to preserve Formoterol diluted, i.e. as it would be necessary for its distribution on the market.

An object of the present invention is to make a preparation containing Formoterol as an active substance, so that the preparation remains stable in time for a sufficiently extended period so that a commercial use is allowed.

Another object of the invention is to allow a preparation containing Formoterol as an active substance to be delivered very efficiently by an electric nebulizer for respiratory topical administration.

Therefore, according to a first aspect of the present invention, there is provided a pharmaceutical formulation of a Formoterol-containing water-propylene glycol solution, having the following characteristics:
- Formoterol, as an active substance, in a concentration variable between 0,001 and 0,06 per cent;
- a stabilizer, composed of citric acid and sodium citrate, in an amount variable between 0,005 and 0,5 per cent;
- a solvent of propylene glycol and water, in the required amount to achieve 100 per cent of a finished solution.

According to a second aspect of the present invention, there is provided a method of preparing a Formoterol-containing water-propylene glycol solution comprising the following steps:
- dissolving Formoterol in a suitable reactor in a required amount of propylene glycol by stirring, in order to obtain a propylene glycol solution;
- dissolving citric acid and sodium citrate in a suitable reservoir in 80 per cent of the required water, in order to obtain an aqueous solution;
- transferring the aqueous solution to the propylene glycol solution by stirring to obtain a sole solution;
- determining the pH of the sole solution and using, if any, citric acid at 20 per cent to adjust it until that a value of 5 ± 0.5, and, more particularly, a value of 5.0 ± 0.1 is obtained;
- diluting to volume to obtain a finished solution;
- filtering the finished solution;
- final packaging.

An advantage of the invention is that the solution obtained remains unalterated in time for a sufficiently extended period so that a commercial use is allowed. The solution allows the active substance to be administered constantly in time by a nebulizer.

An embodiment of the method of preparing a Formoterol-containing water-propylene glycol solution according to the present invention is explained below.

In a suitable reactor, such an amount of Formoterol, so that a concentration variable between 0.001 and 0.06 per cent, for example 0.003 per cent, will be obtained in a final solution, is dissolved in the required amount of propylene glycol by stirring, in order to obtain a propylene glycol solution.

A stabilizer comprising of citric acid and sodium citrate is dissolved in a suitable reservoir in 80 per cent of the required water, in order to obtain an aqueous solution. The amount of stabilizer is between 0.005 and 0.5 per cent, e.g. 0.05 per cent. The mutual rate of propylene glycol to water is variable from 10/90 to 90/10, in the required amount to achieve 100 per cent of a finished solution.

Then, the aqueous solution is transferred to the propylene glycol solution to obtain a sole solution.

The pH of this sole solution is determined. If the pH is not the desired one, citric acid at 20 per cent is used to adjust it.

The sole, water-propylene glycol solution, is diluted to volume to obtain the finished solution.

The finished solution is filtered by a filter with a 0.22 µm wide mesh and then is packaged in containers which are, preferably but not exclusively, monodose 2 ml phials made of amber-coloured, neutral glass.

Researches on the stability are carried out with the laboratories of the inventors following the ICH (International Conference on Harmonisation) guide lines, on the grounds of which it is proved that a typical Formoterol fumarate-containing water-propylene glycol solution with a concentration of 30 micrograms per ml, in phials made of amber-coloured, neutral glass and sealed in both their ends, remains unaltered at 5 Celsius degrees in a period up to 20 months. Once at room temperature this solution remains stable in a further period of 6 months. Yet, the tolerability of the inhalation solution appeared good, and the respirable portion was equal to 6.6 micrograms.

The nebulization efficiency by the electrical nebulizer is more than 30 per cent after 5 minutes.

## Claims

1. A pharmaceutical formulation of a Formoterol-containing water-propylene glycol solution, **characterized by** the following composition:
- Formoterol, as an active substance, in a concentration variable between 0,001 and 0,06 per cent;
- a stabilizer, composed of citric acid and sodium citrate, in an amount variable between 0,005 and 0,5 per cent;
- a solvent of propylene glycol and water, in the required amount to achieve 100 per cent of a finished solution.

2. The pharmaceutical formulation according to claim 1, **characterized in that** Formoterol is in a concentration of 0,003 per cent with respect to the finished solution.

3. The pharmaceutical formulation according to claim 1, **characterized in that** the stabilizer is in a concentration of 0,05 per cent with respect to the finished solution.

4. The pharmaceutical formulation according to claim 1, **characterized in that** the mutual rate of propylene glycol to water in the solvent is variable from 10/90 to 90/10.

5. A method of preparing a Formoterol-containing water-propylene glycol solution according to claim 1, **characterized in that** the method comprises the following steps:
- dissolving Formoterol in a suitable reactor in a required amount of propylene glycol by stirring, in order to obtain a propylene glycol solution;
- dissolving citric acid and sodium citrate in a suitable reservoir in 80 per cent of the required water, in order to obtain an aqueous solution;
- transferring the aqueous solution to the propylene glycol solution to obtain a sole solution;
- determining the pH of the sole solution and using, if any, citric acid at 20 per cent to adjust it until that a value of 5 ± 0.5, and, more particularly, a value of 5.0 ± 0.1 is obtained;
- diluting to volume to obtain a finished solution;
- filtering the finished solution;
- final packaging.

6. The method according to claim 5, **characterized in that** final packaging is in containers.

7. The method according to claim 6, **characterized in that** said containers are monodose 2 ml phials made of amber-coloured, neutral glass.
